# EUROPÄISCHE PATENTSCHRIFT

(11) **EP 3 588 175 B1**
(45) Veröffentlichungstag und Bekanntmachung des Hinweises auf die Patenterteilung: **04.11.2020**
(21) Anmeldenummer: 19189087.0
(22) Anmeldetag: 25.01.2018
(51) Int. Cl.: G02C 13/00, G03B 17/48

(54) **VORRICHTUNG ZUR BESTIMMUNG VON ZENTRIERPARAMETERN FÜR DIE BRILLENANPASSUNG**
DEVICE FOR DETERMINING CENTRING PARAMETERS FOR SPECTACLE ADAPTATION
DISPOSITIF DE DÉTERMINATION DES PARAMÈTRES DE CENTRAGE POUR L'ADAPTATION DE LUNETTES

(30) Priorität: 27.01.2017 EP 17153556
(43) Veröffentlichungstag der Anmeldung: 01.01.2020
(62) Teilanmeldung aus: 18704453.2
(73) Patentinhaber: Carl Zeiss Vision International GmbH, 73430 Aalen (DE); Carl Zeiss AG, 73447 Oberkochen (DE)
(72) Erfinder: ALVAREZ DIEZ, Cristina, 73447 Oberkochen (DE); BREUNINGER, Tobias, 72585 Riederich (DE); GAMPERLING, Michael, 89340 Leipheim (DE); SCHWARZ, Oliver, 73479 Ellwangen (DE); WIDULLE, Frank, 89233 Neu-Ulm (DE)
(74) Vertreter: Patentanwälte Bregenzer und Reule Partnerschaftsgesellschaft mbB

(56) Entgegenhaltungen:
- US-A1- 2003 081 173
- US-A1- 2007 285 528
- US-A1- 2010 239 135
- US-B1- 9 395 562

## Beschreibung

Die Erfindung betrifft eine Vorrichtung zur Bestimmung von Zentrierparametern für die Brillenanpassung gemäß Oberbegriff des Anspruchs 1.

Zentrierparameter werden benutzt, um Brillengläser korrekt in einer Brillenfassung anzuordnen bzw. zu zentrieren, so dass die Brillengläser in korrekter Position relativ zu den Augen der die Brille tragenden Person angeordnet sind. Dabei handelt es sich zum Teil um anatomische Parameter der betreffenden Person, wie beispielsweise den Pupillenabstand, zum Teil um rein fassungsspezifische Parameter wie die Fassungsscheibenbreite oder die Fassungsscheibenhöhe und zum Teil um Kombinationen aus anatomischen und fassungsspezifischen Parametern, wie beispielsweise den Hornhautscheitelabstand und die Durchblickshöhe. Einen Überblick über die gängigen Zentrierparameter gibt die DIN EN ISO 13666 vom Oktober 2013.

Es sind Vorrichtungen bekannt, mit denen eine automatische Bestimmung von Zentrierparametern möglich ist. So ist beispielsweise aus der EP 1 844 363 B2 eine solche Vorrichtung bekannt, bei der mittels zweier Kameras aus unterschiedlichen Blickrichtungen Bilder einer eine Brille bzw. Brillenfassung tragenden Person aufgenommen werden, so dass aus den aufgenommenen Bilddaten Zentrierparameter bestimmt werden können. Aus der US 2010/239135 A1 ist eine Vorrichtung mit den Merkmalen des Oberbegriffs des Anspruches 1 bekannt, die der Untersuchung von Kindern mit Schiefköpfigkeit dient und keine Zentrierparameter bestimmt.

Bei bekannten Vorrichtungen zur Bestimmung von Zentrierparametern besteht jedoch oft das Problem, dass die aus unterschiedlichen Blickrichtungen aufgenommenen Bilder in Ihrer Qualität und Verwertbarkeit schwanken.

Es ist daher Aufgabe der Erfindung, eine Vorrichtung der eingangs genannten Art derart weiterzubilden, dass bessere Bilder der Person, für die die Zentrierparameter bestimmt werden sollen, erzeugt werden können.

Diese Aufgabe wird erfindungsgemäß durch eine Vorrichtung mit den Merkmalen des Anspruches 1 gelöst. Vorteilhafte Weiterbildungen der Erfindung sind Gegenstand der abhängigen Ansprüche.

Des Weiteren umfasst die Erfindung ein Verfahren zur Bestimmung von Zentrierparametern für die Brillenanpassung und ein Computerprogramm mit Programmcode zur Durchführung des Verfahrens.

Ausgehend von der US 2010/239135 löst der Gegenstand des Anspruchs 1 die Aufgabe, einen Probanden besser im Innenraum plazieren zu können.

Erfindungsgemäß weist die Vorrichtung einen Kameraträger auf, der einen Innenraum teilweise umschließt, diesen aber nach oben, nach unten und zu einer Rückseite hin offen lässt. Ein Proband kann sich dann von der Rückseite her in den Innenraum bewegen. Der Innenraum wird durch den Kameraträger nach vorne und zu den Seiten begrenzt. Die Kameras sind auf den Innenraum gerichtet. Sie sind in einer Kameraanordnung angeordnet, die sich zwischen zwei freien Enden des Kameraträgers erstreckt. Vorzugsweise erstreckt sich eine konkave Biegung des Kameraträgers in derselben Richtung, in der sich die Kameraanordnung zwischen den freien Enden erstreckt. Dabei ist es möglich, eine kontinuierliche und konkave Biegung vorzusehen, dem Kameraträger beispielsweise in Draufsicht eine Kreisbogenform zu geben, oder aber einen eckigen Grundriss vorzusehen, so dass der Kameraträger eine Frontpartie und in einem Winkel von der Frontpartie abstehende Seitenpartien aufweist. Die Kameras der Kameraanordnung können sämtlich auf gleicher Höhe in einer Kamerareihe angeordnet oder in ihrer Höhe gegeneinander versetzt sein. Um den Innenraum gut auszuleuchten, weist der Kameraträger eine Beleuchtungseinrichtung auf, die sich vorzugsweise zwischen den freien Enden über eine Länge erstreckt, die mindestens der in einer Umfangsrichtung zwischen den freien Enden gemessenen Länge der Kameraanordnung entspricht. Zweckmäßig weist die Beleuchtungseinrichtung mindestens eine Lichtleiste oder Leuchtmittelreihe mit einer Vielzahl von Leuchtmitteln auf. Die Leuchtmittel sind in einer oder mehreren Reihen angeordnet. Die mindestens eine Lichtleiste mit ihrer Vielzahl von Leuchtmitteln, die vorzugsweise als LEDs ausgebildet sind, ermöglicht eine in hohem Maße gleichmäßige Ausleuchtung des Innenraums, auf den die Kameras gerichtet sind. Es kann vorgesehen sein, dass die Beleuchtungseinrichtung so ausgestaltet ist, dass die auf einer vorbestimmten Oberfläche im Innenraum, nämlich in einem Bereich einer mittig im Innenraum angeordneten Zylindermantelfläche mit 20 cm Durchmesser, der sich über eine Höhe von 20cm und einen Mittelpunktswinkel von 180 Grad erstreckt, gemessene Lichtintensität an jeder Stelle der Oberfläche um maximal +50% und -30% von einem vorgegebenen Basiswert abweicht. Dieser beträgt beispielsweise 1000 Lux, was als ausreichende Ausleuchtung angesehen wird. Dabei kann die Beleuchtungseinrichtung so eingerichtet sein, dass die Lichtintensität an jeder Stelle des vorgegebenen Bereichs einen vorgegebenen Mindestwert überschreitet und dann nicht zwingend insbesondere in Abhängigkeit von Umgebungslicht geregelt werden muss. Die Homogenität des Lichts im Innenraum wird weiter verbessert, wenn die Leuchtmittel ihr Licht durch mindestens ein großflächiges Fenster in den Innenraum emittieren. Reflexionen in den Pupillen sind dann, wenn sie überhaupt auftreten, sehr schwach und stören die Zentriermessung kaum. Alle Leuchtmittel weisen zudem vorzugsweise eine im wesentlichen einheitliche Farbtemperatur von ca. 4000 K +/- 1000 K auf.

Um die Ausleuchtung des Innenraums weiter zu verbessern, wird bevorzugt, dass die Beleuchtungseinrichtung eine erste, über der Kameraanordnung angeordnete, und eine zweite, unter der Kameraanordnung angeordnete Lichtleiste aufweist. Weiter wird bevorzugt, dass die erste und die zweite Lichtleiste jeweils nahe den freien Enden des Kameraträgers durch vertikal verlaufende weitere Lichtleisten miteinander verbunden sind. Die Kameraanordnung wird dann an der Innenfläche des Kameraträgers ringsum durch die Leuchtmittel der Lichtleisten umrahmt, welche sich bevorzugt über einen Mittelpunktswinkel von mindestens 180 Grad erstrecken. Der Abstand der oberen Lichtleiste von der unteren Lichtleiste wird vorzugsweise so gewählt, dass die oben erwähnte Homogenität der Ausleuchtung mit der Abweichung von maximal +50% und -30% vom Basiswert in einem Bereich gegeben ist, der eine Höhe von ca. 20cm hat. Die vertikal verlaufenden weiteren Lichtleisten oder Leuchtmittelreihen erhöhen die Lichtintensität im Bereich der Enden der ersten und der zweiten Lichtleiste oder Leuchtmittelreihe, wo von jenseits dieser Enden kein Lichteinfall kommt.

Um aussagefähige Bilddaten erzeugen zu können, wird bevorzugt, dass sich die Anordnung der Kameras über einen Mittelpunktswinkel von mindestens 150 Grad erstreckt bzw. dass die optischen Achsen der dem freien Ende des Kameraträgers am nächsten gelegenen Kameras einen Winkel von mindestens 150 Grad einschließen. Es ist dann möglich, Bilder des Probanden nicht nur im Wesentlichen von vorn, sondern auch von beiden Seiten aufzunehmen. Zudem wird bevorzugt, dass sich die freien Enden des Kameraträgers in einem Abstand von mindestens 20cm und vorzugsweise von mindestens 25cm zueinander befinden. Ein Proband mit durchschnittlich breitem Kopf kann sich dann bequem von der Rückseite in den Innenraum bewegen.

Zweckmäßig weist die Kameraanordnung eine ungerade Zahl von Kameras auf mit einer mittig angeordneten Frontkamera und bezüglich einer durch die optische Achse der Frontkamera verlaufenden Symmetrieebene symmetrisch angeordneten Seitenkameras. Je mehr Kameras die Kameraanordnung enthält, desto genauer können die Zentrierparameter bestimmt werden.

Vorteilhaft ist eine Steuer- oder Regeleinrichtung zum automatischen oder manuellen Steuern oder Regeln der Lichtintensität der Leuchtmittel in Abhängigkeit von der Helligkeit im Innenraum und vorzugsweise in Abhängigkeit von der mittels der Kameras detektierten Helligkeit vorgesehen. Dabei wird bevorzugt, dass Sektoren der Beleuchtungseinrichtung und/oder einzelne Leuchtmittel getrennt voneinander steuerbar oder regelbar sind. Dadurch wird der Tatsache Rechnung getragen, dass in der Praxis regelmäßig Fremdlicht auf den Probanden einfällt, beispielsweise durch ein Fenster des Raumes, in dem sich die Vorrichtung befindet. Um eine gleichmäßige Ausleuchtung des Probanden zu gewährleisten, kann es dann erforderlich sein, beispielsweise die dem Fenster zugewandte Gesichtshälfte weniger stark, die dem Fenster abgewandte Gesichtshälfte aber umso stärker auszuleuchten.

Die Erfindung sieht einen Abstandssensor vor, der der Messung des Abstands des Kopfes des Probanden zur Mitte des Kameraträgers dient. Zudem ist eine Anzeigeeinheit zur Anzeige des Abstands oder einer den Abstand charakterisierenden Größe vorgesehen. Dadurch wird dem Probanden eine Positionierung am gewünschten Ort, beispielsweise in der Mitte des Innenraums, erleichtert. Während der Proband eine Positionierung in der optischen Achse der in der Mitte des Kameraträgers angeordneten Frontkamera meist selbst hinreichend genau vornehmen kann, kann er den Abstand zur Mitte des Kameraträgers meist nur schlecht abschätzen. Der Abstandssensor ermittelt diesen Abstand und zeigt dem Probanden an, ob er zu weit vorne, zu weit hinten oder richtig steht. Dabei kann ein zusätzlicher Abstandssensor vorgesehen sein, oder einige der Kameras bilden derart den Abstandssensor, dass sie aus unterschiedlichen Winkeln den Probanden aufnehmen und durch ein geeignetes Abstandsmessverfahren den Abstand bestimmen. Die Anzeigeeinheit wird durch die Beleuchtungseinrichtung gebildet. Diese kann so eingerichtet sein, dass sie die Farbe des abgestrahlten Lichts abhängig vom Abstand des Kopfes von der Mitte des Kameraträgers ändert und ausgehend von rein weißem Licht, das eine korrekte Positionierung anzeigt, immer mehr farbiges Licht beimischt. Es ist auch möglich, dass bei falscher Positionierung des Probanden die Beleuchtungseinrichtung durch örtliche Intensitäts- oder Farbänderungen ein Lauflicht erzeugt, das die Richtung anzeigt, in die sich der Proband zur richtigen Positionierung bewegen muss.

Vorteilhaft ist am Kameraträger eine Fixationseinrichtung zum Erzeugen eines Fixationsmusters für den Probanden angeordnet, vorzugsweise eine zumindest ein Specklemuster als Fixationsmuster erzeugende Fixationseinrichtung. Eine solche Fixationseinrichtung ist ausführlich in der EP 1 704 437 B1 beschrieben, auf die in diesem Zusammenhang ausdrücklich Bezug genommen wird.

Zweckmäßig wird mindestens einer der folgenden Zentrierparameter bestimmt: Hornhautscheitelabstand mindestens eines Auges, Durchblickpunkt durch die Ebene des Brillenglases für mindestens ein Auge, Abstand zwischen den Pupillen, Abstand mindestens einer der Pupillen zu einer Symmetrieebene der Brille oder Brillenfassung, Lage der Glasränder bezüglich mindestens einer Pupille, Verkippung der Fassungsebenen zur Vertikalen (pantoskopischer Winkel). Das erfindungsgemäße Verfahren kann dadurch vereinfacht werden, dass die Glasränder durch Boxen angenähert werden, die durch Boxlänge und Boxhöhe definiert werden.

Die erfindungsgemäß bestimmten Zentrierparameter können vorteilhaft zum Zentrieren eines Brillenglases in einer Brillenfassung und/oder zum Einschleifen eines Brillenglases in eine Brillenfassung herangezogen werden. Dabei wird in einem Verfahrensschritt das mindestens eine Brillenglas mit den bestimmten Zentrierparametern in der Brillenfassung zentriert oder es wird das mindestens eine Brillenglas basierend auf den bestimmten Zentrierparametern für eine Anordnung in der Brillenfassung eingeschliffen. Auf diese Weise können Brillengläser und Brillen hergestellt werden.

Im Folgenden wird die Erfindung anhand eines in der Zeichnung schematisch dargestellten Ausführungsbeispiels näher erläutert. Es zeigt:

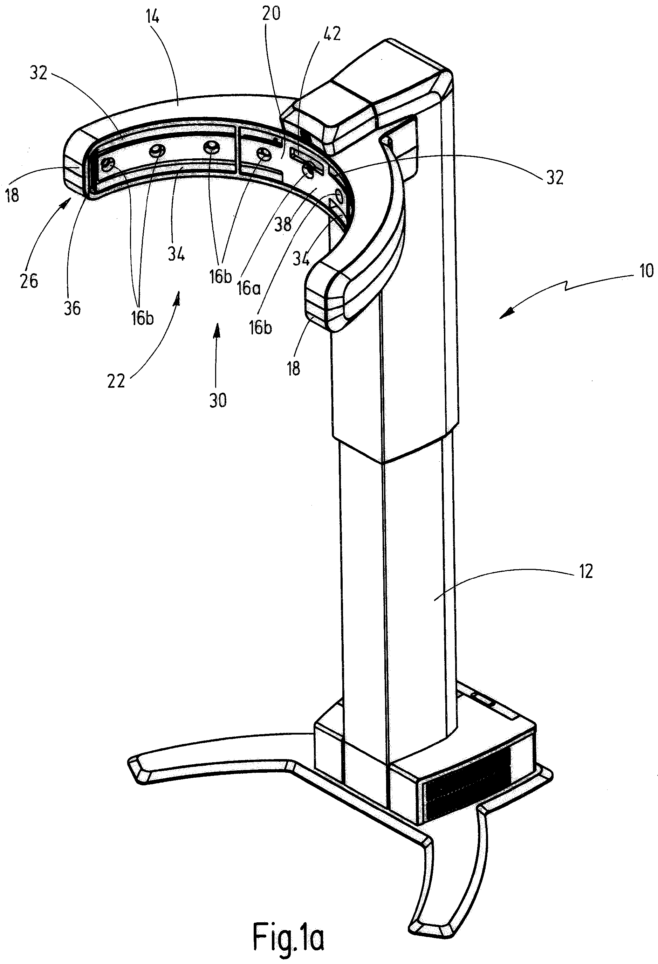

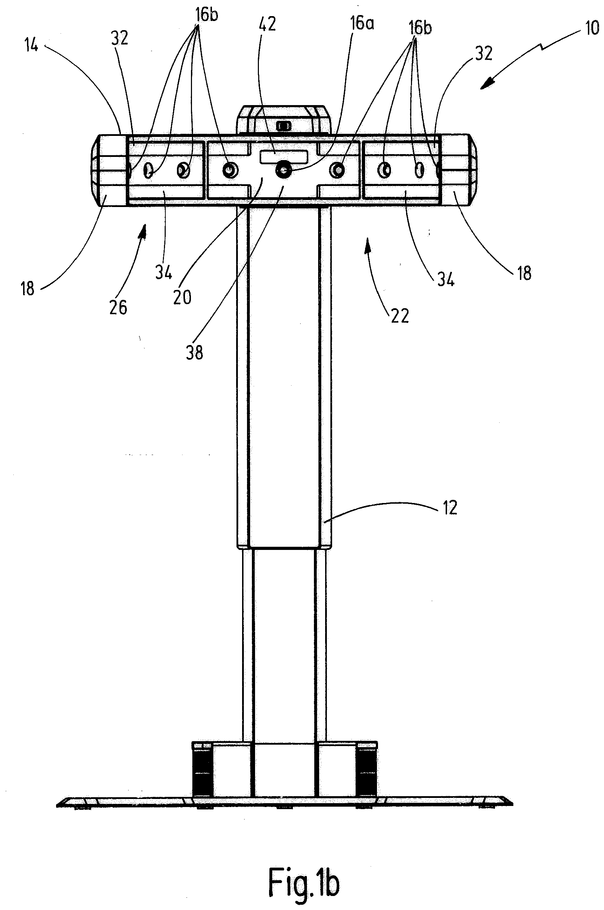

- Figur 1a, b: eine Vorrichtung zur Bestimmung von Zentrierparametern in perspektivischer Ansicht und in einer Ansicht von vorne.

Die in der Zeichnung dargestellte Vorrichtung 10 dient der Bestimmung von Zentrierparametern für die Brillenanpassung. Sie weist eine Säule 12 auf, die höhenverstellbar einen starren Kameraträger 14 trägt, welcher wiederum eine Anzahl Kameras 16a, 16b trägt. Der Kameraträger 14 ist in Draufsicht näherungsweise kreisförmig gebogen und erstreckt sich zwischen zwei freien Enden 18, welche im Abstand zueinander angeordnet sind. Nach vorne, also zur Säule 12 hin, und zu den Seiten umschließt eine Innenfläche 20 des Kameraträgers 14 einen Innenraum 22, in dem sich bei der Aufnahme von Bildern durch die Kameras 16a, 16b der Kopf eines Probanden befindet. Die Innenfläche 20 ist in einer Richtung, die zwischen den freien Enden 18 verläuft, konkav gebogen und weist beispielsweise die Form eines Abschnitts einer Zylindermantelfläche auf, wobei der Zylinder eine kreisrunde oder ovale Grundfläche haben kann. Um den Kameraträger 14 bezüglich des Kopfs des Probanden auf der richtigen Höhe positionieren zu können, ist in der Säule 12 eine nicht näher dargestellte Hubeinrichtung angeordnet, mit der der Kameraträger 14 motorisch angetrieben auf und ab bewegt werden kann.

Alle Kameras 16a, 16b sind äquiangular in einer sich zwischen den freien Enden 18 erstreckenden Kameraanordnung 26 angeordnet. Im vorliegenden Ausführungsbeispiel ist die Kameraanordnung 26 als Kamerareihe 26 ausgebildet, deren Kameras 16a, 16b sich alle in derselben Höhe befinden, wobei ihre optischen Achsen auf den Innenraum 22 gerichtet sind. Im vorliegenden Ausführungsbeispiel umfasst die Kamerareihe 26 eine in der Mitte des Kameraträgers 14 angeordnete Frontkamera 16a, deren optische Achse frontal auf das Gesicht des Probanden gerichtet ist, sowie acht paarweise symmetrisch bezüglich einer durch die optische Achse der Frontkamera 16a verlaufenden senkrechten Symmetrieebene angeordnete Seitenkameras 16b von denen jeweils vier von links und von rechts auf das Gesicht des Probanden gerichtet sind. Die Kameras 16a, 16b sind zudem kalibriert, so dass sie gleichzeitig kalibrierte Bilder des Probanden aufnehmen können. Die Kalibrierung umfasst die extrinsischen Eigenschaften wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird. Eine ausführliche Beschreibung der Kalibrierung von Kameras findet sich im Lehrbuch "Multiple View Geometry in Computer Vision" von Richard Hartley und Andrew Zisserman, 2. Auflage, Cambridge University Press 2004, und dort insbesondere auf Seite 8.

Der Kameraträger 14 umschließt den Innenraum 22 nur nach vorne, zur Säule 12 hin, und zu den Seiten, also links und rechts des Kopfes des Probanden. Nach oben, unten sowie zu einer Rückseite 30 hin ist er offen, wobei die freien Enden 18 zueinander einen Abstand von mindestens 25cm aufweisen, so dass sich der Proband bequem von der Rückseite aus nähern kann. Im gezeigten Ausführungsbeispiel beträgt der Abstand 70 bis 80cm, und die Kameras 16a, 16b sind in gleichen Winkelabständen zueinander angeordnet.

Zur Ausleuchtung des Innenraums 22 ist eine Beleuchtungseinrichtung mit einer oberhalb der Kamerareihe 26 verlaufenden oberen Lichtleiste 32 sowie einer unterhalb der Kamerareihe 26 verlaufenden unteren Lichtleiste 34 vorgesehen, welche jeweils eine Vielzahl von LEDs als Leuchtmittel aufweisen. Die obere Lichtleiste 32 und die untere Lichtleiste 34 erstrecken sich jeweils durchgehend oder mit Unterbrechungen über eine Länge, die mindestens so groß ist wie die Länge der in Umfangsrichtung zwischen den freien Enden 18 gemessenen Länge der Kamerareihe 26. Diese entspricht einem Umfangswinkel von mindestens 160 Grad. Nahe den freien Enden 18 sind die obere Lichtleiste 32 und die untere Lichtleiste 34 jeweils mittels einer vertikal verlaufenden weiteren Lichtleiste 36 miteinander verbunden. Die Kamerareihe 26 wird somit vollständig durch mindestens eine Reihe LEDs umrahmt. Die Vorrichtung 10 weist zudem eine in der Zeichnung nicht näher dargestellte Steuer- oder Regeleinrichtung auf, mit der die von den LEDs abgestrahlte Lichtintensität abhängig von der durch die Kameras 16a, 16b detektierten Lichtintensität gesteuert oder geregelt werden kann. Die LEDs der Lichtleisten 32, 34, 36 sind dabei zu Sektoren zusammengefasst, deren abgestrahlte Lichtintensitäten getrennt voneinander gesteuert bzw. geregelt werden können. Zudem ist vorgesehen, dass auch die von den einzelnen LEDs abgestrahlten Lichtintensitäten mittels der Steuer- oder Regeleinrichtung getrennt voneinander gesteuert oder geregelt werden können.

Um den Probanden richtig im Innenraum 22 positionieren zu können, sind die beiden der Frontkamera 16a nächstgelegenen Seitenkameras 16b dazu eingerichtet, den Abstand des Kopfs des Probanden von der Mitte 38 des Kameraträgers 14 zu messen. Mittels einer nicht näher dargestellten Anzeigeeinheit wird dem Probanden angezeigt, ob er richtig steht oder nicht. Die Anzeigeeinheit weist mehrere unterschiedlich eingefärbte Lichtquellen auf, die in einer Reihe angeordnet sind. Die mittlere Lichtquelle leuchtet grün, wenn der Proband richtig steht. Ausgehend von der mittleren Lichtquelle gibt es in jeder Richtung in dieser Reihenfolge eine gelbe, eine orangene und eine rote Lichtquelle, die entsprechend der Farbe anzeigt, wenn der Proband ein wenig, deutlich oder viel zu weit von der Mitte 38 des Kameraträgers 14 entfernt bzw. ein wenig, deutlich oder viel zu nah zur Mitte 38 steht. Um bei der Bestimmung der Zentrierparameter sicherzustellen, dass die Blickrichtung des Probanden ins Unendliche gerichtet ist, ist eine am Kameraträger 14 angeordnete Fixationseinrichtung 42 vorgesehen, die ein Fixationsmuster für den Probanden in Form eines Specklemusters erzeugt. Das Fixationsmuster ist etwas höher angeordnet als die Frontkamera 16a, so dass der Proband über diese hinwegblickt. Damit kann sein Gesicht im größtmöglichen Umfang aufgenommen werden.

Die Vorrichtung 10 eignet sich insbesondere auch zur Herstellung eines Avatars des Kopfs des Probanden, welcher zur Bestimmung der Zentrierparameter herangezogen werden kann. Zum diesem Zweck werden durch die Kameras 16a, 16b kalibrierte Bilder des Kopfs des Probanden ohne Brille bzw. Brillenfassung aufgenommen. Dabei ist es vorteilhaft, wenn die Bilder gleichzeitig aufgenommen werden. Mittels eines geeigneten Prozesses zur geometrischen Positionsbestimmung wie beispielsweise Triangulation, Stereo-Rekonstruktion, Multiview-Reconstruction oder Structure from Motion wird ein dreidimensionales Tiefenprofil des Kopfes erstellt, das diesen näherungsweise sehr gut abbildet. Der Kopf wird durch eine Vielzahl von Punkten abgebildet, die mittels eines Netzmusters miteinander verbunden werden können oder aber als Punktwolke gespeichert werden können. Bei der anschließenden Bestimmung der Zentrierparameter kann der so ermittelte Avatar herangezogen werden, um Zentrierparameter zu bestimmen, die aufgrund der geometrischen Eigenschaften der Brille bzw. Brillenfassung, die der Proband trägt, nicht oder nur näherungsweise bestimmt werden können. Beispielsweise kann ein breiter Fassungsbügel das Auge in einer Seitenaufnahme soweit verdecken, dass der Hornhautscheitelabstand nicht oder nur sehr ungenau bestimmt werden kann. Zudem können gefärbte oder stark spiegelnde Gläser die Augen nicht oder nur sehr schlecht erkennen lassen. Um dem zu begegnen, wird auf die von den Kameras 16a, 16b aufgenommenen Bilder des die Brille oder Brillenfassung tragenden Probanden das Tiefenprofil des Avatars projiziert und die Zentrierparameter, die aufgrund der durch die Brille bzw. Brillenfassung eingeschränkten Sicht nur ungenügend bestimmt werden können, werden mittels der Bilddaten des Avatars bestimmt. Dabei kann eine Anpassung des Avatars auf die Bilder des die Brille bzw. Brillenfassung tragenden Probanden zur Minimierung von Abweichungen erfolgen. Zudem kann der Avatar dazu herangezogen werden, virtuell Brillen bzw. Brillenfassungen anzuprobieren, indem die ihre Geometrie beschreibenden Daten auf den Avatar eingeblendet werden.

## Patentansprüche

1. Vorrichtung zur Bestimmung von Zentrierparametern für die Brillenanpassung mit einem Kameraträger (14), welcher einen nach oben, nach unten und zu einer Rückseite (30) hin offenen Innenraum (22) teilweise umschließt und mindestens drei Kameras (16a, 16b) trägt, die zwischen zwei freien Enden (18) des Kameraträgers (14) angeordnet und auf den Innenraum (22) gerichtet sind, und wobei der Kameraträger (14) zur Beleuchtung des Innenraums (22) eine Beleuchtungseinrichtung (32, 34, 36) aufweist, **gekennzeichnet durch** einen Abstandssensor zur Messung des Abstands eines in dem Innenraum (22) angeordneten Kopfes eines Probanden zur Mitte des Kameraträgers (14) sowie eine Anzeigeeinheit zur Anzeige des Abstands oder einer den Abstand charakterisierenden Größe, wobei die Beleuchtungseinrichtung (32, 34, 36) die Anzeigeeinheit bildet.

2. Vorrichtung nach Anspruch 1, **dadurch gekennzeichnet, dass** die Beleuchtungseinrichtung (32, 34, 36) eingerichtet ist, die Farbe des von ihr abgestrahlten Lichts abhängig vom Abstand des Kopfes von der Mitte des Kameraträgers (14) zu ändern und/oder ein bewegtes Lichtmuster zu erzeugen.

3. Vorrichtung nach einem der vorangehenden Ansprüche, **dadurch gekennzeichnet, dass** die Kameras (16a, 16b) eingerichtet sind, aus unterschiedlichen Perspektiven erste Bilder des Kopfs eines Probanden sowie mindestens ein zweites Bild des eine Brille oder eine Brillenfassung tragenden Kopfs aufzunehmen und dass die Vorrichtung (10) eine Recheneinrichtung aufweist, welche eingerichtet ist, mittels eines Prozesses der geometrischen Positionsbestimmung aus den ersten Bildern ein dreidimensionales Tiefenprofil zumindest eines Teils des Kopfs zu bestimmen und zu speichern, das Tiefenprofil auf das mindestens eine zweite Bild zu projizieren und Zentrierparameter aus Bilddaten des mindestens einen zweiten Bilds und aus Daten des auf das mindestens eine zweite Bild projizierten Tiefenprofils zu bestimmen.

4. Verfahren zur Bestimmung von Zentrierparametern, für die Brillenanpassung wobei ein Kopf eines eine Brille tragenden Probanden aus mindestens drei Aufnahmerichtungen aufgenommen wird und wobei der Kopf des Probanden mittels einer Beleuchtungseinrichtung (32, 34, 36) beleuchtet wird, **dadurch gekennzeichnet, dass** ein Ort des Kopfes bezüglich der Aufnahmerichtungen gemessen und eine den Ort des Kopfes charakterisierende Größe angezeigt wird und dass der Abstand oder die den Abstand charakterisierende Größe mittels der Beleuchtungseinrichtung (32, 34, 36) angezeigt wird.

5. Verfahren nach Anspruch 4, **dadurch gekennzeichnet, dass** der Abstand oder die den Abstand charakterisierende Größe mittels der Farbe des von der Beleuchtungseinrichtung (32, 34, 36) abgestrahlten Lichts angezeigt wird.

6. Verfahren nach einem der Ansprüche 4 oder 5, **dadurch gekennzeichnet, dass** mindestens drei kalibrierte, aus unterschiedlichen Perspektiven aufgenommene erste Bilder des Kopfs eines Probanden ohne Brille bereitgestellt werden und dass mittels eines Prozesses zur geometrischen Positionsbestimmung aus den ersten Bildern ein dreidimensionales Tiefenprofil zumindest eines Teils des Kopfs bestimmt und gespeichert wird.

7. Verfahren nach Anspruch 6, **dadurch gekennzeichnet, dass** mindestens ein zweites Bild des eine Brille oder eine Brillenfassung tragenden Kopfs aufgenommen wird, wobei das Tiefenprofil auf das mindestens eine zweite Bild projiziert wird und wobei Zentrierparameter aus Bilddaten des mindestens einen zweiten Bilds und aus Daten des auf das mindestens eine zweite Bild projizierten Tiefenprofils bestimmt werden.

8. Verfahren nach Anspruch 7, **dadurch gekennzeichnet, dass** die Projektion des Tiefenprofils auf das mindestens eine zweite Bild mittels eines Optimierungsprozesses zur Minimierung von Abweichungen an das mindestens eine zweite Bild angepasst wird.

9. Verfahren nach einem der Ansprüche 6 bis 7, **dadurch gekennzeichnet, dass** zur geometrischen Positionsbestimmung ein Triangulationsverfahren verwendet wird.

10. Verfahren nach einem der Ansprüche 4 bis 9, **dadurch gekennzeichnet, dass** mindestens einer der folgenden Zentrierparameter bestimmt wird: Hornhautscheitelabstand mindestens eines Auges, Durchblickpunkt durch die Ebene des Brillenglases für mindestens ein Auge, Abstand zwischen den Pupillen, Abstand mindestens einer der Pupillen zu einer Symmetrieebene der Brille oder Brillenfassung, Lage der Glasränder bezüglich mindestens einer Pupille, Verkippung der Fassungsebenen zur Vertikalen (pantoskopischer Winkel).

11. Verfahren nach Anspruch 10, **dadurch gekennzeichnet, dass** die Glasränder durch Boxen angenähert werden, die durch Boxlänge und Boxhöhe definiert werden.

12. Computerprogramm mit Programmcode zur Durchführung aller Verfahrensschritte nach einem der Ansprüche 4 bis 11, wenn das Computerprogramm in einem Computer geladen und/oder in einem Computer ausgeführt wird.

13. Verwendung einer Vorrichtung (10) nach einem der Ansprüche 1 bis 3 zur Durchführung des Verfahrens nach einem der Ansprüche 4 bis 11.

14. Verfahren zum Zentrieren mindestens eines Brillenglases in einer Brillenfassung, **dadurch gekennzeichnet, dass** in einem ersten Verfahrensschritt Zentrierparameter mit einem Verfahren nach einem der Ansprüche 4 bis 11 bestimmt werden und dass in einem zweiten Verfahrensschritt das mindestens eine Brillenglas mit den im ersten Verfahrensschritt bestimmten Zentrierparametern in der Brillenfassung zentriert wird.

15. Verfahren zum Einschleifen mindestens eines Brillenglases in eine Brillenfassung, **dadurch gekennzeichnet, dass** in einem ersten Verfahrensschritt Zentrierparameter mit einem Verfahren nach einem der Ansprüche 4 bis 11 bestimmt werden und dass in einem zweiten Verfahrensschritt das mindestens eine Brillenglas basierend auf den im ersten Verfahrensschritt bestimmten Zentrierparametern für eine Anordnung in der Brillenfassung eingeschliffen wird

16. Verfahren zum Herstellen eines Brillenglases, **gekennzeichnet durch** den Verfahrensschritt Einschleifen des Brillenglases in eine Brillenfassung nach dem Verfahren gemäß Anspruch 15.

17. Verfahren zum Herstellen einer Brille, **dadurch gekennzeichnet, dass** ein Verfahren nach einem der Ansprüche 14 bis 16 verwendet wird.

## Claims

1. Apparatus for determining centration parameters for the adjustment of spectacles, comprising a camera carrier (14), which partly encloses an interior (22) that is open to the top, to the bottom and to a rear side (30) and which carries at least three cameras (16a, 16b) which are arranged between two free ends (18) of the camera carrier (14) and directed onto the interior (22), and wherein the camera carrier (14) has an illumination device (32, 34, 36) for illuminating the interior (22), **characterized by** a distance sensor for measuring the distance of a head of a subject that is arranged in the interior (22) from the centre of the camera carrier (14) and an indication unit for indicating the distance or a variable characterizing the distance, wherein the illumination device (32, 34, 36) forms the indication unit.

2. Apparatus according to Claim 1, **characterized in that** the illumination device (32, 34, 36) is set up to change the colour of the light emitted thereby and/or produce a chasing light pattern in dependence on the distance of the head from the centre of the camera carrier (14) .

3. Apparatus as claimed in either of the preceding claims, **characterized in that** the cameras (16a, 16b) are set up to record from different perspectives first images of the head of a subject and at least one second image of the head wearing spectacles or a spectacle frame and **in that** the apparatus (10) has a computational device that is set up to determine a three-dimensional depth profile of at least part of the head from the first images using a process for a geometric position determination and to store it, to project the depth profile onto the at least one second image and to determine centration parameters from image data of the at least one second image and from data of the depth profile projected onto the at least one second image.

4. Method for determining centration parameters for the adjustment of spectacles, wherein the head of a subject wearing spectacles is recorded from at least three recording directions and wherein the head of the subject is illuminated by means of an illumination device (32, 34, 36), **characterized in that** a location of the head relative to the recording directions is measured and a variable characterizing the location of the head is indicated and **in that** the distance or the variable characterizing the distance is indicated by means of the illumination device (32, 34, 36).

5. Method according to Claim 4, **characterized in that** the distance or the variable characterizing the distance is indicated by means of the colour of the light emitted by the illumination device (32, 34, 36).

6. Method according to either of Claims 4 and 5, **characterized in that** at least three calibrated first images of the head of a subject without spectacles, recorded from different perspectives, are provided and **in that** a three-dimensional depth profile of at least part of the head is determined from the first images using a process for a geometric position determination and is stored.

7. Method according to Claim 6, **characterized in that** at least one second image of the head wearing spectacles or a spectacle frame is recorded, wherein the depth profile is projected onto the at least one second image and wherein centration parameters are determined from image data of the at least one second image and from data of the depth profile projected onto the at least one second image.

8. Method according to Claim 7, **characterized in that** the projection of the depth profile onto the at least one second image is adapted to the at least one second image by way of an optimization process for minimizing deviations.

9. Method according to one of Claims 6 to 7, **characterized in that** a triangulation method is used for a geometric position determination.

10. Method according to one of Claims 4 to 9, **characterized in that** at least one of the following centration parameters is determined: vertex distance of at least one eye, visual point through the plane of the spectacle lens for at least one eye, distance between the pupils, distance of at least one of the pupils from a plane of symmetry of the spectacles or the spectacle frame, location of the lens rims with respect to at least one pupil, tilt of the frame planes with respect to the vertical (pantoscopic angle).

11. Method according to Claim 10, **characterized in that** the spectacle rims are approximated by boxes defined by a box length and a box height.

12. Computer program having program code for carrying out all method steps according to one of Claims 4 to 11 when the computer program is loaded on a computer and/or executed on a computer.

13. Use of an apparatus (10) according to one of Claims 1 to 3 for carrying out the method according to one of Claims 4 to 11.

14. Method for centring at least one spectacle lens in a spectacle frame, **characterized in that** centration parameters are determined in a first method step using a method according to one of Claims 4 to 11, and **in that** the at least one spectacle lens is centred with the centration parameters determined in the first method step in the spectacle frame in a second method step.

15. Method for grinding at least one spectacle lens into a spectacle frame, **characterized in that** centration parameters are determined in a first method step using a method according to one of Claims 4 to 11, and **in that**, in a second method step, the at least one spectacle lens is ground for an arrangement in the spectacle frame on the basis of the centration parameters determined in the first method step.

16. Method for producing a spectacle lens, **characterized by** the method step of grinding the spectacle lens into a spectacle frame according to the method according to Claim 15.

17. Method for producing a spectacle lens, **characterized in that** a method according to one of Claims 14 to 16 is used.

## Revendications

1. Dispositif permettant de déterminer des paramètres de centrage pour l'adaptation de lunettes, comprenant un support de caméra (14) qui entoure en partie un espace intérieur (22) ouvert vers le haut, vers le bas et vers une face arrière (30) et porte au moins trois caméras (16a, 16b) qui sont disposées entre deux extrémités libres (18) du support de caméra (14) et sont orientées vers l'espace intérieur (22), et dans lequel le support de caméra (14) présente un dispositif d'éclairage (32, 34, 36) pour éclairer l'espace intérieur (22), **caractérisé par** un capteur de distance pour mesurer une distance de la tête d'un sujet, disposée dans l'espace intérieur (22), jusqu'au centre du support de caméra (14), ainsi que par une unité d'affichage permettant d'afficher la distance ou une grandeur caractérisant la distance, le dispositif d'éclairage (32, 34, 36) constituant l'unité d'affichage.

2. Dispositif selon la revendication 1, **caractérisé en ce que** le dispositif d'éclairage (32, 34, 36) est aménagé pour modifier la couleur de la lumière qu'il émet en fonction de la distance de la tête par rapport au centre du support de caméra (14) et/ou pour produire un motif lumineux animé.

3. Dispositif selon l'une quelconque des revendications précédentes, **caractérisé en ce que** les caméras (16a, 16b) sont aménagées pour prendre des premières images de la tête d'un sujet sous différentes perspectives ainsi qu'au moins une deuxième image de la tête portant des lunettes ou une monture de lunettes, et **en ce que** le dispositif (10) présente un dispositif de calcul qui est aménagé pour déterminer et enregistrer au moyen d'un processus de détermination de position géométrique à partir des premières images un profil de profondeur tridimensionnel d'au moins une partie de la tête, pour projeter le profil de profondeur sur ladite au moins une deuxième image et pour déterminer des paramètres de centrage à partir de données d'image de ladite au moins une deuxième image et à partir de données du profil de profondeur projeté sur ladite au moins une deuxième image.

4. Procédé de détermination de paramètres de centrage pour l'adaptation de lunettes, la tête d'un sujet portant des lunettes étant imagée à partir d'au moins trois directions de prise de vue, et la tête du sujet étant éclairée au moyen d'un dispositif d'éclairage (32, 34, 36), **caractérisé en ce qu'**un emplacement de la tête est mesuré par rapport aux directions de prise de vue et une grandeur caractérisant l'emplacement de la tête est affichée, et **en ce que** la distance ou la grandeur caractérisant la distance est affichée au moyen du dispositif d'éclairage (32, 34, 36).

5. Procédé selon la revendication 4, **caractérisé en ce que** la distance ou la grandeur caractérisant la distance est affichée au moyen de la couleur de la lumière émise par le dispositif d'éclairage (32, 34, 36) .

6. Procédé selon l'une quelconque des revendications 4 ou 5, **caractérisé en ce qu'**au moins trois premières images calibrées, prises sous différentes perspectives, de la tête d'un sujet sans lunettes sont fournies, et **en ce qu'**un processus de détermination de position géométrique permet à partir des premières images de déterminer et d'enregistrer un profil de profondeur tridimensionnel au moins d'une partie de la tête.

7. Procédé selon la revendication 6, **caractérisé en ce qu'**au moins une deuxième image de la tête portant des lunettes ou une monture de lunettes est prise, le profil de profondeur étant projeté sur ladite au moins une deuxième image, et des paramètres de centrage étant déterminés à partir de données d'image de ladite au moins une deuxième image et à partir de données du profil de profondeur projeté sur ladite au moins une deuxième image.

8. Procédé selon la revendication 7, **caractérisé en ce que** la projection du profil de profondeur sur ladite au moins une deuxième image est adaptée au moyen d'un processus d'optimisation pour minimiser des écarts.

9. Procédé selon l'une quelconque des revendications 6 à 7, **caractérisé en ce qu'**un procédé de triangulation est utilisé pour la détermination de position géométrique.

10. Procédé selon l'une quelconque des revendications 4 à 9, **caractérisé en ce qu'**au moins l'un des paramètres de centrage suivants est déterminé : la distance œil-verre d'au moins un œil, le point de vision à travers le plan du verre de lunettes pour au moins un œil, l'écartement entre les pupilles, la distance d'au moins l'une des pupilles par rapport à un plan de symétrie des lunettes ou de la monture de lunettes, la position des bords de verre par rapport à au moins une pupille, l'inclinaison des plans de monture par rapport à la verticale (angle pantoscopique).

11. Procédé selon la revendication 10, **caractérisé en ce que** les bords de verre sont rendus approximativement par des boîtes qui sont définies par une longueur de boîte et une hauteur de boîte.

12. Programme informatique, comprenant du code programme pour exécuter toutes les étapes de procédé selon l'une quelconque des revendications 4 à 11 lorsque le programme informatique est chargé sur un ordinateur et/ou exécuté sur un ordinateur.

13. Utilisation d'un dispositif (10) selon l'une quelconque des revendications 1 à 3 pour exécuter le procédé selon l'une quelconque des revendications 4 à 11.

14. Procédé de centrage d'au moins un verre de lunettes dans une monture de lunettes, **caractérisé en ce que** dans une première étape de procédé, des paramètres de centrage sont déterminés par un procédé selon l'une quelconque des revendications 4 à 11, et **en ce que** dans une deuxième étape de procédé, ledit au moins un verre de lunettes est centré dans la monture de lunettes à l'aide des paramètres de centrage déterminés à la première étape de procédé.

15. Procédé d'insertion d'au moins un verre de lunettes dans une monture de lunettes, **caractérisé en ce que** dans une première étape de procédé, des paramètres de centrage sont déterminés par un procédé selon l'une quelconque des revendications 4 à 11, et **en ce que** dans une deuxième étape de procédé, ledit au moins un verre de lunettes est inséré dans la monture de lunettes sur la base des paramètres de centrage pour un agencement, déterminés à la première étape de procédé.

16. Procédé de fabrication d'un verre de lunettes, **caractérisé par** l'étape de procédé consistant à insérer le verre de lunettes dans une monture de lunettes selon le procédé selon la revendication 15.

17. Procédé de fabrication d'une paire de lunettes, **caractérisé en ce qu'**un procédé selon l'une quelconque des revendications 14 à 16 est utilisé.
